# EUROPEAN PATENT APPLICATION

(11) **EP 2 636 673 A1**
(43) Date of publication of application: **11.09.2013**
(21) Application number: 12158253.0
(22) Date of filing: 06.03.2012
(51) Int. Cl.: C07D 403/04, C07D 403/14, C07D 417/04, C07D 417/14, A61K 31/4184, A61K 31/428, A61K 31/4439, A61K 31/4436, A61P 35/00

(54) **Aminomethylene pyrazolones with therapeutic activity**

(71) Applicant: Compound Handling B.V., 2629 HT Delft (NL)
(72) Inventor: Van Hooij, Onno, 6500 HB Nijmegen (NL); Schalken, Jacobus Antonius, 6524 LH Nijmegen (NL); Viëtor, Hendrik Engelbertus, 1404 AK Bussum (NL); Piet, Dennis Patrick, 2904 Br Capelle aan den Ijssel (NL); Maas , Petrus Emmanuel Marie, 2629 Delft (NL); Tijhuis, Johann Heinrich, 2629 Ht Delft (NL); Deerenberg, Sirik, 2629 Ht Delft (NL); Sprenkels, Nanda Elisabeth, 2629 Ht Delft (NL); Tang, Siu Ha, 2492 PB Den Haag (NL)
(74) Representative: Habets, Winand

(57) **Abstract**

The invention provides for a compound having the structure according to formula I 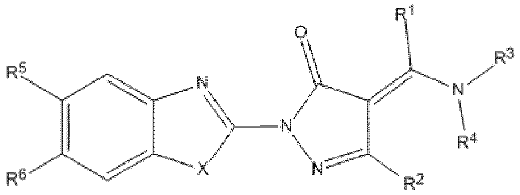 wherein:
X is NH or S; R¹ is H or (1C-4C)alkyl; R² is (1C-4C)alkyl, phenyl or a monocyclic aromatic ring having one or more N-, O- or S- atoms in the ring, which alkyl, phenyl or aromatic ring is optionally substituted with one or more groups selected from (1C-4C)alkyl, (1C-4C)alkyloxy, halo(1C-4C)alkyl, halo(1C-4C)alkyloxy, phenyloxy , phenylthio, halogen, or nitro; R³ and R⁴ are each independently H, (1C-6C)alkyl, (2C-6C) alkenyl, (2C-6C)alkynyl, cyano, (3C-6C)cycloalkyl, phenyl, a monocyclic aromatic ring having one or more N-, O- or S- atoms in the ring, a monocyclic non-aromatic ring having one or more N-, O- or S- atoms in the ring, each optionally further substituted with hydroxyl, (1C-4C)alkoxy, phenyl, cycloalkyl, piperidyl, piperazinyl, furyl, thienyl, pirazinyl, pyrrolyl, 2H-pyrrolyl, pyrazolyl, isoxazolyl, isothiazolyl, pyrrolidonyl, pyrrolinyl, imidazolinyl, imidazolyl, a monocyclic aromatic ring having one or more N-, O- or S-atoms in the ring, whereby each of these optional substituents is optionally further substituted with (1 C-4C)alkyl, (1 C-4C)alkyloxy, halo(1 C-4C)alkyl, halo(1 C-4C)alkyloxy, halogen, nitro or (1 C-2C)dioxol forming a ring; or R³ and R⁴ form together pyrrolyl, imidazolyl, pyrazolyl, pyrrolidinyl, pyrrolinylimidazolidinyl, imidazolinyl, piperidyl, piperazinylmorpholinyl, each optionally substituted with (1 C-6C)alkyl, phenyl(1 C-4C)alkyl, phenylketo(1C-4C)alkyl; R⁵ is H or CF₃; R⁶ is (1 C-4C)alkyl, (1 C-4C)alkyloxy, halo(1 C-4C)alkyl, halo(1 C-4C)alkyloxy, nitro or halogen; for use in treatments of carcinoma, in particular to delay, prevent or reverse metastasis in prostate cancer.

## Description

### Field of the invention

The invention is in the field of medicinal treatment of carcinoma, in particular by using an aminomethylene pyrazolone.

### Background of the invention

Carcinomas, which are cancers that originate from epithelial tissues, comprise the most dangerous types of cancers. Gastric, bladder and esophageal cancer are examples of carcinomas of epithelial origin. Glandular tissue often is of epithelial origin, so that also breast cancer, prostate cancer and pancreas cancer also belong to the group of cancers from epithelial origin.

If a carcinoma is diagnosed early and still localized, the disease is curable by surgery, radiation therapy with or without (neo)adjuvant and chances of survival are high (>90%). However, in early stages, cancers can grow slowly and can remain locally confined for many years without causing overt symptoms. Notorious in this respect is prostate cancer. Therefore, such types of cancer often remain undiagnosed until cancerous cells have already spread beyond the prostate into the surrounding tissues (local spread) or eventually migrate (metastasize) through the blood stream or lymphatic spread into other areas of the body.

Progressive growth of epithelial cancer and invasive metastasis involves a multistep process. Tumors can generally not grow beyond a certain size, due to a lack of oxygen and other essential nutrients. However, tumors induce blood vessel growth by secreting various growth factors which induce capillary growth into the tumor to supply nutrients, allowing for tumor expansion. This physiological process is called angiogenesis. Angiogenesis is a normal and vital process in growth and development, such as in wound healing, but also a fundamental step in the transition of tumors from small harmless clusters of cells to a malignant tumor. Angiogenesis is also required for the spread, or metastasis, of a tumor. Single cancer cells can break away from an established solid tumor, enter the blood vessel, and be carried to a distant site, where they can implant and begin the growth of a secondary tumor. Such spread to other tissues (metastasis) involves invasion of other parts of the body by mesenchymal cells. Cancer cell invasion and spread is determined by epithelial-mesenchymal-transition (EMT). The spread to other tissues is preceded by transition of the epithelial cells to mesenchymal cells, indicated as epithelial-mesenchymal transition (EMT). Thereby the incipient cancer cells acquire mesenchymal, fibroblast-like properties and show reduced intercellular adhesion and increased motility, endowing the incipient cancer cells with invasive and metastatic properties. The reversed process in which mesenchymal-to-epithelial transition (MET), creates new secondary tumors at the other sites. Many patients die when diagnosed with an aggressive form of cancer in which the cancerous cells have spread, or metastasized.

It is important to improve the efficacy of medicinal treatment by providing compounds which can interfere with the metastasis of cells, more in particular compounds that can reverse EMT or interfere with the process of EMT.

Some treatment options of carcinomas are available, but are of limited success and provide no permanent cure. For prostate or breast cancer endocrine therapy, also called hormone deprivation therapy, has long been considered as the main suppression therapy to control neoplasms. The goal is to limit the body's production of the hormones. However, current endocrine therapy does not cure prostate or breast cancer. Moreover, it has become clear that expansive growth of cancer cells that become unresponsive (resistant) to the current available endocrine therapies is inevitable. In addition, it was found that in the majority of advanced cancers the hormone receptor mediated signaling pathway is still active, even at extremely low hormone levels. At this stage, the cancer can no longer be treated with available therapy and often results in progression to a lethal disease.

New chemotherapeutic drugs demonstrating improved response rates and prolonged survival are being developed. One of the examples is docetaxel (Taxotere). Unfortunately, chemotherapy reaches all parts of the body, not just only the cancer cells. It has been established that these therapies have serious side effects. Patients will undergo low blood cell counts, nausea, vomiting, abdominal pain, diarrhea, hair loss, impotence, incontinence and other unwanted symptoms. Hence, the side effects significantly hamper the quality of life of the patients. Many scientists are convinced that this treatment will offer little room for future improvements and has come close to the end of its product life cycle. Docetaxel is the current standard of care for patients that are unresponsive to the currently available endocrine therapies. In view of limited curative potential of docetaxel, and also in view of better understanding of the underlying etiology of the disease and improved early diagnosis, there is an urgent need for novel treatment strategies to prevent the progression, treat the tumor and avoid metastasis of this disease.

### Summary of the invention

The present invention provides for compounds having the structure according to formula I 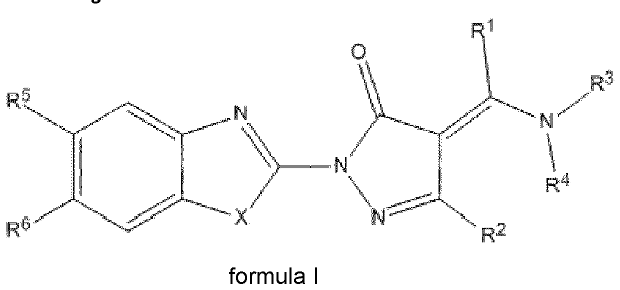 wherein:
X is NH or S;
R¹ is H or (1C-4C)alkyl;
R² is (1C-4C)alkyl, phenyl or a monocyclic aromatic ring having one or more N-, O- or S- atoms in the ring, which alkyl, phenyl or aromatic ring is optionally substituted with one or more groups selected from (1 C-4C)alkyl, (1 C-4C)alkyloxy, halo(1 C-4C)alkyl, halo(1 C-4C)alkyloxy, phenyloxy , phenylthio, halogen, or nitro;
R³ and R⁴ are each independently H, (1C-6C)alkyl, (2C-6C) alkenyl, (2C-6C)alkynyl, cyano, (3C-6C)cycloalkyl, phenyl, a monocyclic aromatic ring having one or more N-, O- or S- atoms in the ring, a monocyclic non-aromatic ring having one or more N-, O- or S- atoms in the ring, each optionally substituted with hydroxyl, (1 C-4C)alkoxy, phenyl, cycloalkyl, piperidyl, piperazinyl, furyl, thienyl, pirazinyl, pyrrolyl, 2H-pyrrolyl, pyrazolyl, isoxazolyl, isothiazolyl, pyrrolidonyl, pyrrolinyl, imidazolinyl, imidazolyl, a monocyclic aromatic ring having one or more N-, O- or S- atoms in the ring, whereby each of these optional substituents is optionally further substituted with (1 C-4C)alkyl, (1 C-4C)alkyloxy, halo(1 C-4C)alkyl, halo(1 C-4C)alkyloxy, halogen, nitro or (1C-2C)dioxol forming a ring; or
R³ and R⁴ form together pyrrolyl, imidazolyl, pyrazolyl, pyrrolidinyl, pyrrolinylimidazolidinyl, imidazolinyl, piperidyl, piperazinylmorpholinyl, each optionally substituted with (1 C-6C)alkyl, phenyl(1 C-4C)alkyl, phenylketo(1 C-4C)alkyl;
R⁵ is H or CF3;
R⁶ is (1 C-4C)alkyl, (1 C-4C)alkyloxy, halo(1 C-4C)alkyl, halo(1 C-4C)alkyloxy, nitro or halogen;
   and pharmaceutically acceptable addition salts thereof.

Such compounds may advantageously be used for therapy, i.e. the prevention or treatment of a disease. More in particular they may be used in the prevention or treatment of a carcinoma. Even more in particular the compounds according to the invention may be used in the treatment or prevention of metastasis of a carcinoma.

The term carcinoma is used herein to indicate a cancer of epithelial origin, more in particular a disease selected from the group consisting of gastric cancer, bladder cancer, esophageal cancer, breast cancer, prostate cancer or pancreas cancer. In particular the treatment or prevention of metastasis of prostate cancer is preferred.

In a more specific embodiment, the invention is directed to a compound having the structure and meanings of symbols according to formula I and wherein R³ and R⁴ are independently hydrogen, methyl, ethyl, or propyl or a group as represented in the following list of structures: 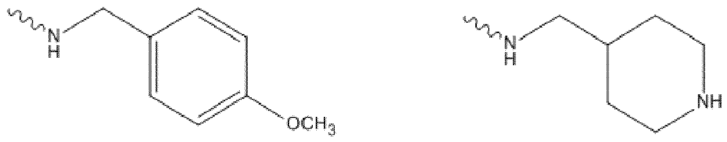 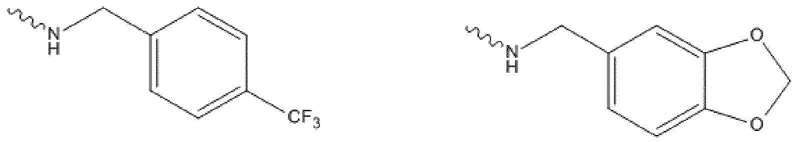 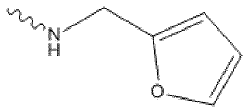 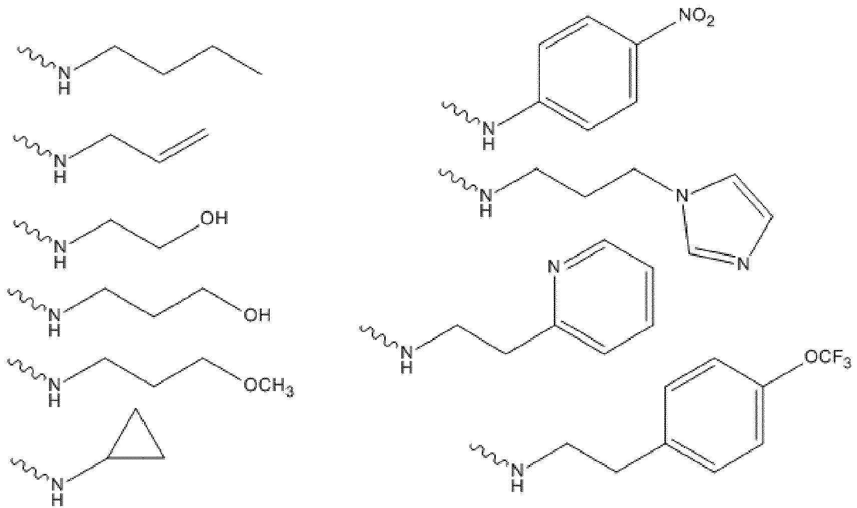 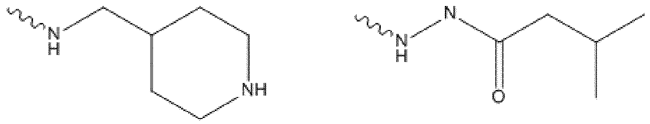 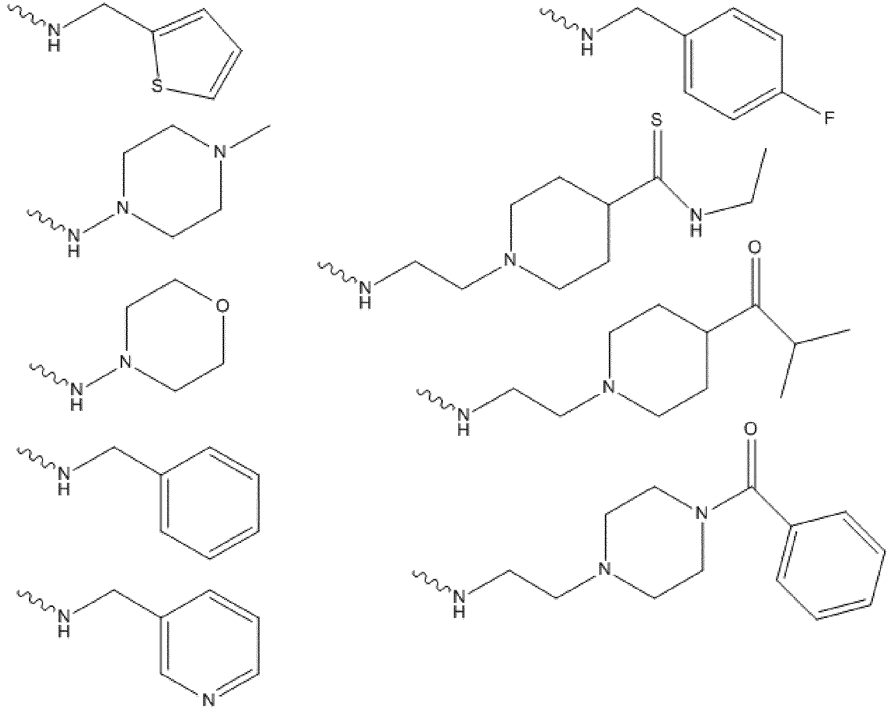 or R³ ad R⁴ form together an optionally substituted ring as represented in the following structures

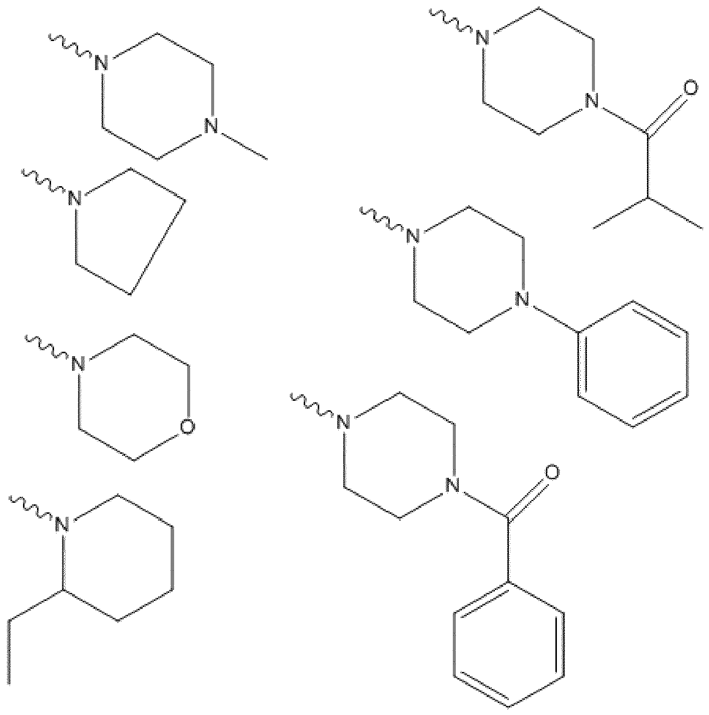

Another embodiment of the invention is a compound having the structure according to formula I wherein
R¹ is H or (1C-4C)alkyl
R² is (1C-4C)alkyl, phenyl or a monocyclic aromatic ring having one or more N-, O- or S- atoms in the ring, which alkyl, phenyl or aromatic ring is optionally substituted with one or more groups selected from (1C-4C)alkyl, OCF3 or halogen; R⁵ and R⁶ are hydrogen; or a pharmaceutically acceptable addition salt thereof.

Preferred embodiments of the invention are as those defined above but wherein the meaning of X is S.

Other preferred embodiments are those as defined above, wherein R¹ is H or (1C-4C)alkyl and wherein R² is(1C-4C)alkyl or phenyl.

Other preferred embodiments are the embodiments as defined above wherein R³ and R⁴ are both methyl or wherein R³ is hydrogen and R⁴ is as defined above.

Another preferred embodiment of the invention is a compound according to formula II: 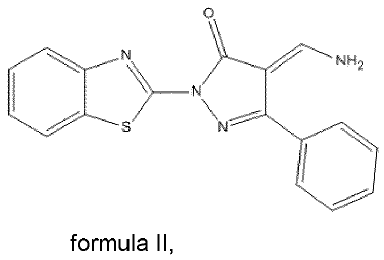 or a pharmaceutically acceptable addition salt thereof.

### Detailed description of the invention

The terms used in the description have the following meaning:
The prefix (1 C-4C) refers to the number of 1-4 carbon atoms in the alkyl, alkenyl or alkynyl group. The definition includes amongst others a methyl, ethyl, propyl, isopropyl, butyl, tertiairy butyl, vinyl, ethynyl, cyclopropyl and propynyl..

Halo or halogen means fluorine, chlorine, bromine, iodine.

A pharmaceutically acceptable addition salt is known in the art of pharmaceutics, such as a chloride, maleate, lactate etc.

It should be realized that the compounds according to the invention exist in tautomeric isomers when R³ and/or R⁴ are hydrogen. As shown in the formulas A, B and C the double bond system over the aminomethylenepyrazolone [A] can shift to the iminomethylpyrazolone system in [B] so that the delocalized representation as in formula [C] would be an equivalent manner to represent the compounds according to the invention. Anyway, these tautomeric isomers are comprised into the definition of the compounds according to the invention as defined with the support of the formula I.

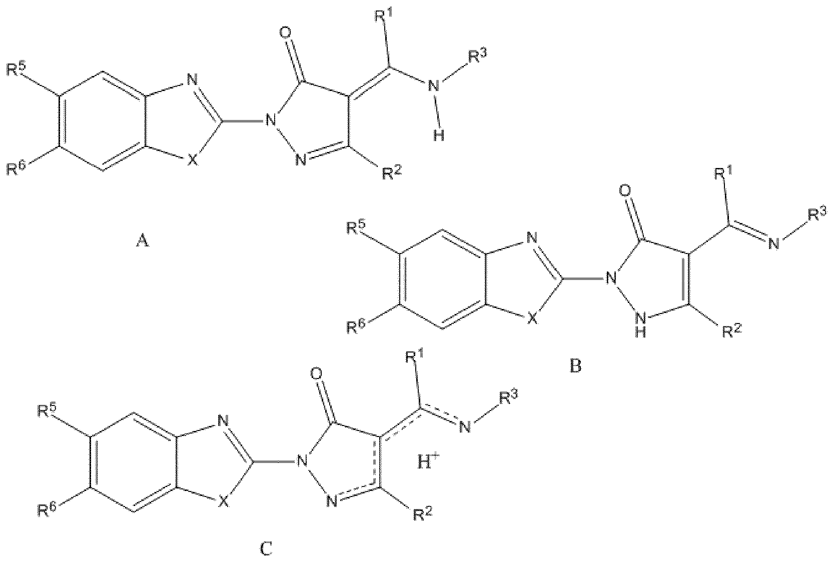

A compound according to the invention may be prepared, for example, by starting with preparation a 2,4-dihydropyrazol-3-one scaffold, which is synthesized through a condensation-cyclization reaction of suitable hydrazines and acetoacetate esters in either ethanol or ethanol / acetic acid mixtures at reflux temperatures where X=S and in methanol containing a catalytic amount of concentrated HCl where X=N. The cyclization product is usually collected by filtration, rinsing of the filter cake with ethanol and in vacuo drying.

In the second reaction step, the thus obtained 1,2-dihydropyrazol-3-one is subjected to an aminomethylenation reaction in THF at room temperature. The precipitated product may be purified by filtration, rinsing of the filter cake with a suitable solvent and in vacuo drying.

In the third and final step, the aminomethylidenepyrazol-3-one is treated with a suitable primary amine in methanol or ethanol at room temperature or at any temperature leading up to reflux temperature of the reaction solvent. The product may be purified by filtration and rinsing with methanol or ethanol and in vacuo drying.

A pharmaceutically acceptable addition salt of a compound may be prepared according to conventional methods. Salts are usually obtained by combining the free base with inorganic or organic acids such as hydrochloric, fumaric, maleic, citric or succinic acid.

The therapeutic or preventive effect of a compound according to the invention can be obtained by administration of the compound to a patient (human or animal, male or female) in need of treatment by administering the compound either topically, locally or systemically. Any enteral or parenteral route, such as transdermal, transmucosal, oral, rectal, intravenous, intramuscular or subcutaneous, can be selected as most suitable under the circumstances of the condition of the patient and the location of cancer cells. The administration will be greatly aided by the manufacture of pharmaceutical compositions comprising a compound according to the invention. A pharmaceutical formulation of a compound according to the invention can be prepared according to methods known in the art, varying from conventional pills, tablets and solutions to more sophisticated formulations for depot formulations or formulations adapted for particular routes of administration. Resorption of the compound according to the invention by the patient can be facilitated or delayed by pharmaceutical additives.

In therapeutic use it is possible to select particular regimes of administration for continuous or multiple dosing per day, or for detailed treatment regimes for a certain period of time, for example a week, a month or other continuous or intermittent periods. In the field of cancer therapy it is often needed or beneficial to use more than one method to combat the disease. A compound according to the invention is suitable for combination treatment with other treatments.

Dose selection depends on routes of administration and type and condition of the treated patient. The effective dose will usually be in the range of 0.001-1000 mg per patient, or, expressed in amount per kg patient, in particular in consideration of small weight patients (for example children or animals) between 0.0001 - 100 mg/kg. The preferred range is 0.01 - 5 mg/kg or 1- 350 mg for an average human patient

Without wanting to be bound by theory in the use of the invention, it was found that an important contribution to the therapeutic mechanism of compounds of the invention can reside in interference with the process of invasion into healthy tissue, as for example the interaction between prostate cancer cells and the bone micro-environment.

For determining the effectiveness of the compounds according to the invention, we employed a model assay based on the migration of cells in a migration chamber. This model is accepted in the art as providing representative data on the ability of cells to metastasize.

We found that a preferred compound according to the invention inhibits tumor cell invasion more than 25% (+). A particularly preferred compound also showed dose-dependent anti-invasion activity of over 40% (++). The compounds according to the invention are thus capable of interfering with the acquisition of an invasive phenotype in human prostate cancer by inhibiting the EMT process

| Compound | Result |
|---|---|
| 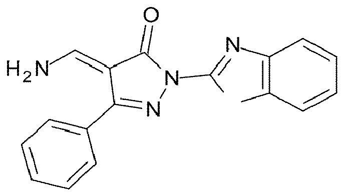 | ++. |
| 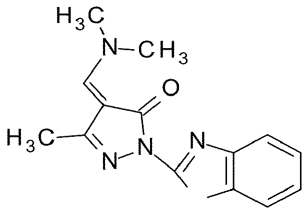 | ++ |
| 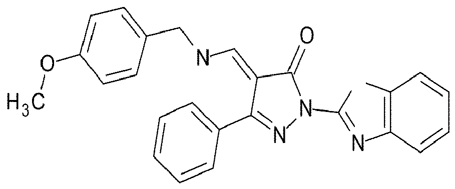 | + |
| 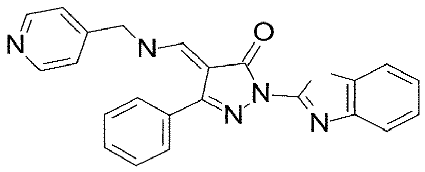 | + |
| 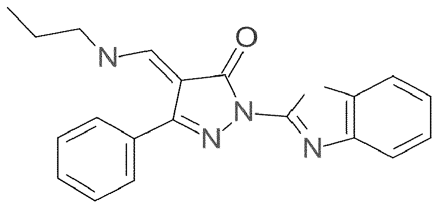 | ++ |
| 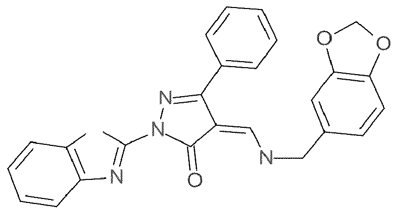 | + |
| 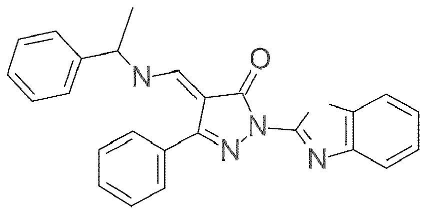 | ++ |
| 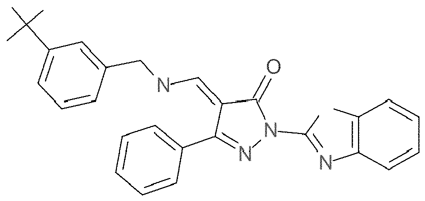 | ++ |

Treatment of mice with 4-[1-Aminomethylidene]-2-benzothiazol-2-yl-5-phenyl-2,4-dihydro-pyrazol-3-one decreased the number of bone lesions and metastatic tumor burden. Cancer cells and tumor burden were monitored by whole body bioluminescence imaging. The data show that the compound according to the invention affects the formation of de novo skeletal metastases by PC-3M-Pro4luc+ cells in vivo.

### Examples

### Example 1: preparation of 4-[1-Aminomethylidene]-2-benzothiazol-2-yl-5-(2-methoxyphenyl)-2,4-dihydropyrazol-3-one.

A solution of 1.00g (4.45mmol) of ethyl (2-methoxybenzoyl)acetate and 743mg (4.45mmol) of 2-hydrazinobenzothiazole in 15ml of ethanol, containing a few drops of AcOH, was refluxed over night under a nitrogen atmosphere. After evaporating the reaction solvent and replacing it with diethyl ether containing a small amount of acetone, the precipitate was filtered, washed with diethyl ether and dried to give 1.33g (4.11 mmol, 92%) of 2-benzothiazol-2-yl-5-(2-methoxyphenyl)-1,2-dihydropyrazol-3-one. 1 H-NMR (DMSO-d6): δ 12.40 (bs, 1 H), 8.05 (d, 1 H), 7.90 (d, 1 H), 7.80 (s, 1 H), 7.50 (m, 2H), 7.40 (t, 1 H), 7.20 (d, 1 H), 7.10 (m, 1 H), 6.05 (s, 1 H), 3.90 (s, 3H).

To a solution of 722mg (2.23mmol) of 2-benzothiazol-2-yl-5-(2-methoxyphenyl)-1,2-dihydropyrazol-3-one in 15ml of THF was added N,N-dimethylformamide dimethylacetal (326μl, 2.46mmol). The reaction was stirred over night at room temperature under a nitrogen atmosphere after which the reaction mixture was diluted with a small amount of diethyl ether. The solids were filtered off, washed with diethyl ether and dried to give 824mg (2.18mmol, 98%) of 2-benzothiazol-2-yl-4-[1-dimethylaminomethylidene]-5-(2-methoxy-phenyl)-2,4-dihydropyrazol-3-one. 1 H-NMR (DMSO-d6): δ 8.00 (d, 1 H), 7.75 (d, 1 H), 7.50 (t, 1 H), 7.40 (m, 2H), 7.30 (m, 2H), 7.20 (d, 1 H), 7.10 (t, 1 H), 3.80 (s, 3H), 3.70 (s, 3H), 3.35 (s, 3H).

A suspension of 625mg (1.65mmol) of 2-benzothiazol-2-yl-4-[1-dimethylaminomethylidene]-5-(2-methoxy-phenyl)-2,4-dihydropyrazol-3-one in 10ml ethanol and 10ml of a 25% ammonia solution was heated to 60°C under a nitrogen atmosphere over night. After cooling to room temperature, the reaction mixture was diluted with a little water, the solids were filtered, washed with ethanol and dried to give 481 mg (1.37mmol, 83%) of 4-[1-aminomethylidene]-2-benzothiazol-2-yl-5-(2-methoxyphenyl)-2,4-dihydropyrazol-3-one. 1 H-NMR (DMSO-d6): δ 9.40 (bs, 2H), 8.00 (d, 1 H), 7.80 (d, 1 H), 7.70 (s, 1 H), 7.45 (m, 3H), 7.30 (t, 1 H), 7.20 (d, 1 H), 7.10 (t, 1 H), 3.80 (s, 3H).

### Example 2: Preparation of -4-[1-Aminomethylidene]-2-benzothiazol-2-yl-5-phenyl-2,4 dihydro-pyrazol-3-one.

A solution of 1.75g (9.08mmol) of ethyl benzoylacetate and 1.50g (9.08mmol) of 2-hydrazinobenzothiazole in 30ml of ethanol was refluxed for 4h under a nitrogen atmosphere. After cooling to room temperature, the precipitate was filtered, washed with cold ethanol, diethylether and dried to give 1.66g (5.66mmol, 62%) of 2-benzothiazol-2-yl-5-phenyl-1,2-dihydropyrazol-3-one as a white solid. ¹H-NMR (DMSO-d6): δ 12.90 (bs, 1 H), 8.05 (d, 1 H), 7.90 (m, 3H), 7.50 (m, 4H), 7.30 (t, 1 H), 6.10 (s, 1 H).

To a solution of 190mg (0.648mmol) of 2-benzothiazol-2-yl-5-phenyl-1,2-dihydropyrazol-3-one in 10ml of THF was added N,N-dimethylformamide dimethylacetal (90μl, 0.680mmol). The reaction was stirred for 3h at room temperature under a nitrogen atmosphere, the solids were filtered off, washed with acetone and dried to give 125mg (0.359mmol, 55%) of 2-benzothiazol-2-yl-4-[1-dimethylaminomethylidene]-5-phenyl-2,4-dihydropyrazol-3-one as a yellow solid. ¹H-NMR (DMSO-d6): δ 8.00 (d, 1 H), 7.80 (d, 1 H), 7.70 (s, 1 H), 7.65 (m, 2H), 7.55 (m, 3H), 7.40 (t, 1 H), 7.30 (t, 1 H), 3.75 (s, 3H), 3.40 (s, 3H).

A suspension of 100mg (0.287mmol) of 2-benzothiazol-2-yl-4-[1-dimethylaminomethylidene]-5-phenyl-2,4-dihydropyrazol-3-one in 5ml of a 25% ammonia solution was heated to 120°C in a pressure vessel overnight. After cooling to room temperature, the solids were filtered, washed with water and dried to give 48mg (0.150mmol, 52%) of 4-[1-aminomethylidene]-2-benzothiazol-2-yl-5-phenyl-2,4-dihydro-pyrazol-3-one as a yellow solid. ¹H-NMR (DMSO-d6): δ 9.05 (bs, 2H), 8.00 (m, 2H), 7.85 (d, 1 H), 7.75 (m, 2H), 7.55 (m, 3H), 7.45 (t, 1 H), 7.30 (t, 1 H).

### Example 3: Preparation of 4-[1-Aminomethylidene]-2-(1H-benzoimidazol-2-yl)-5-phenyl-2,4-dihydropyrazol-3-one

A solution of 500mg (3.37mmol) of 2-hydrazino-1 H-benzimidazole and 713mg (3.70mmol) of ethyl benzoylacetate of in 15ml of methanol containing a catalytic amount of concentrated HCl. The reaction mixture was stirred at 65°C under a nitrogen atmosphere over night. After cooling to room temperature, the precipitate was filtered and dried to give 966mg (3.09mmol, 92%) of 2-(1H-benzoimidazol-2-yl)-5-phenyl-1,2-dihydropyrazol-3-one as the hydrochloride salt. 1H-NMR (DMSO-d6): δ 7.95 (m, 2H), 7.65 (m, 2H), 7.45 (m, 3H), 7.20 (m 2H), 6.10 (s, 1 H).

To a suspension of 100mg (0.36mmol) of 2-(1H-benzoimidazol-2-yl)-5-phenyl-1,2-dihydropyrazol-3-one hydrochloride salt in 5ml of dioxane was added N,N-dimethylformamide dimethylacetal (52μl, 0.39mmol). The reaction was stirred for 2h at room temperature under a nitrogen atmosphere after which the reaction mixture was cooled on an ice bath and diluted with a small amount of diethyl ether. The solids were filtered off, washed with diethyl ether and dried to give 99mg (0.30mmol, 83%) of 2-(1H-benzoimidazol-2-yl)-4-[1-dimethylaminomethylidene]-5-phenyl-2,4-dihydropyrazol-3-one as a yellow solid. 1 H-NMR (DMSO-d6): δ 13.40 (bs, 1 H), 8.55 (bs, 2H), 7.70 (s, 1 H), 7.65 (m, 2H), 7.55 (m, 3H), 7.20 (m, 2H), 3.70 (s, 3H), 3.40 (s, 3H).

A suspension of 50mg (0.15mmol) of 2-(1H-benzoimidazol-2-yl)-4-[1-dimethylaminomethylidene]-5-phenyl-2,4-dihydropyrazol-3-one in 3ml of a 25% ammonia solution was heated to 65°C under a nitrogen atmosphere for 3h. After cooling to room temperature, the solids were filtered and dried to give 32mg (0.11 mmol, 70%) of 4-[1-aminomethylidene]-2-(1 H-benzoimidazol-2-yl)-5-phenyl-2,4-dihydropyrazol-3-one as a yellow solid. 1 H-NMR (DMSO-d6): δ 12.20 (bs, 1 H), 9.40 (bs, 2H), 8.05 (m, 1 H), 7.70 (m, 2H), 7.50 (m, 5H), 7.10 (m, 2H).

### Example 4: Biological methods

The PC-3 prostate cancer cell line (ATCC# CRL-1435) was maintained in RPMI-1640 medium (Invitrogen, 31870), supplemented with 10% Fetal Bovine Serum (Sigma, F7524), L-Glutamine (Invitrogen 25030-024). Cells were split once a week at a 1:10 ratio.

### Example 5: Cell Invasion Assay

For cell invasion assays, PC3 cells were incubated in the presence of a compound according to the invention(10 uM) for 4 days, prior to the invasion assay. Forty thousand cells were seeded into BD Biocoat Matrigel Invasion chambers (8 micron; BD 354480) in serum-free medium. The invasion chamber was placed in a 24-well containing medium with 10% fetal calf serum as chemo-attractant. As a control, the same amount of cells was seeded in 24-well culture plates. After 48 hours incubation, cells in the invasion chamber were removed by aspiration and cleaning the inner compartment with a cotton swab. The invasion chamber was then put into CellTiter-GLO (CTG, Promega-G7571) cell viability reagent, incubated for 15 minutes, and then analyzed on a Victor3 luminometer. Cell invasion was calculated as the CTG activity on the lower part of the membrane divided by the CTG activity of the cells grown in a 24 well plate. Inhibition of cell invasion by a specific compound was estimated by comparing the amount of cell invasion of compound-treated cells versus DMSO treated cells.

## Claims

1. A compound having the structure according to formula I 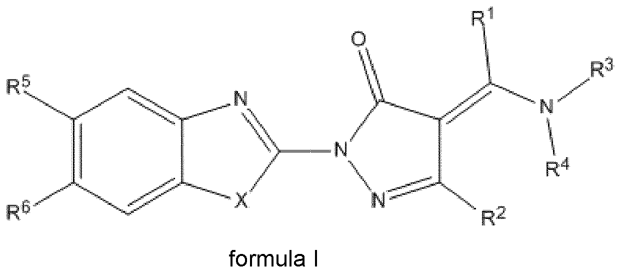 wherein:
X is NH or S;
R¹ is H or (1C-4C)alkyl;
R² is (1C-4C)alkyl, phenyl or a monocyclic aromatic ring having one or more N-, O- or
S- atoms in the ring, which alkyl, phenyl or aromatic ring is optionally substituted with one or more groups selected from (1C-4C)alkyl, (1C-4C)alkyloxy, halo(1C-4C)alkyl, halo(1C-4C)alkyloxy, phenyloxy , phenylthio, halogen, or nitro;
R³ and R⁴ are each independently H, (1C-6C)alkyl, (2C-6C) alkenyl, (2C-6C)alkynyl, cyano, (3C-6C)cycloalkyl, phenyl, a monocyclic aromatic ring having one or more N-,
O- or S- atoms in the ring, a monocyclic non-aromatic ring having one or more N-, O-or S- atoms in the ring, each optionally substituted with hydroxyl, (1 C-4C)alkoxy, phenyl, cycloalkyl, piperidyl, piperazinyl, furyl, thienyl, pirazinyl, pyrrolyl, 2H-pyrrolyl, pyrazolyl, isoxazolyl, isothiazolyl, pyrrolidonyl, pyrrolinyl, imidazolinyl, imidazolyl, a monocyclic aromatic ring having one or more N-, O- or S- atoms in the ring, whereby each of these optional substituents is optionally further substituted with (1 C-4C)alkyl, (1 C-4C)alkyloxy, halo(1 C-4C)alkyl, halo(1C-4C)alkyloxy, halogen, nitro or (1 C-2C)dioxol forming a ring;
or R³ and R⁴ form together pyrrolyl, imidazolyl, pyrazolyl, pyrrolidinyl, pyrrolinylimidazolidinyl, imidazolinyl, piperidyl, piperazinylmorpholinyl, each optionally substituted with (1 C-6C)alkyl, phenyl(1 C-4C)alkyl, phenylketo(1 C-4C)alkyl;
R⁵ is H or CF₃;
R⁶ is (1C-4C)alkyl, (1C-4C)alkyloxy, halo(1C-4C)alkyl, halo(1C-4C)alkyloxy, nitro or halogen;
and pharmaceutically acceptable addition salts thereof.

2. The compound according to claim 1, wherein R³ and R⁴ each independently represent hydrogen, methyl, ethyl or propyl or a group as represented in the following list of structures: 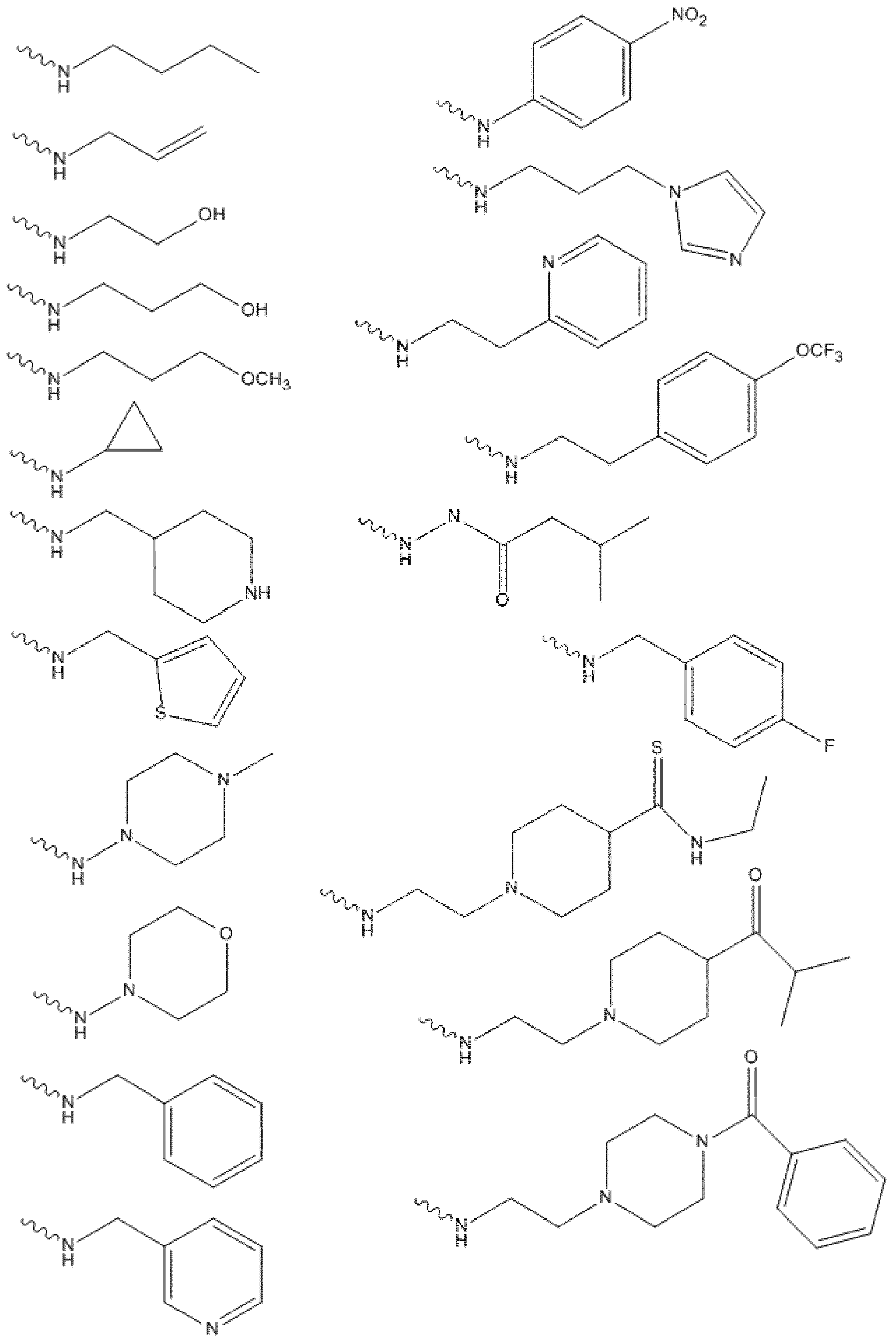 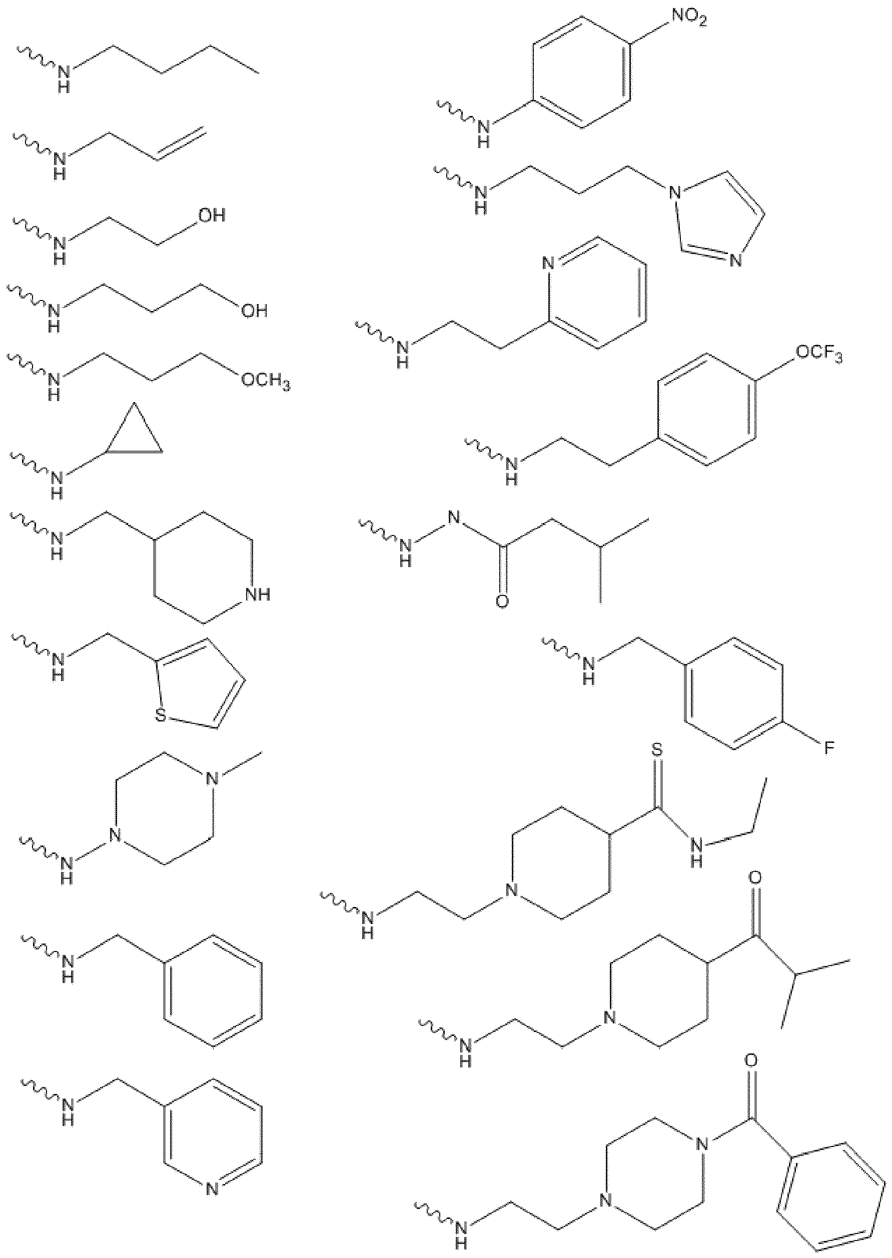 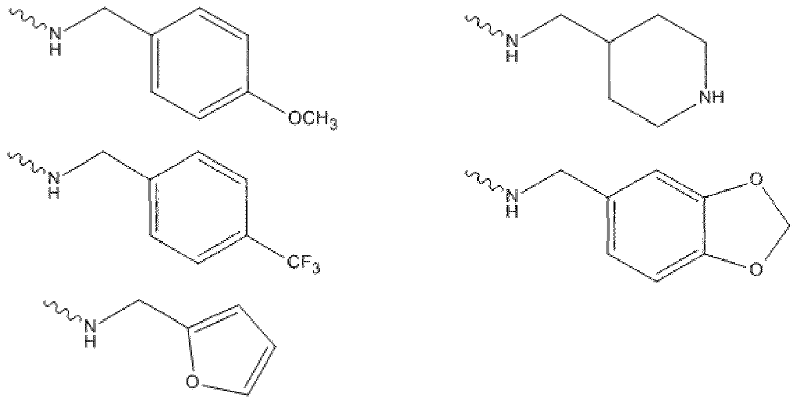 or R³ ad R⁴ form together an optionally substituted ring as represented in the following structures

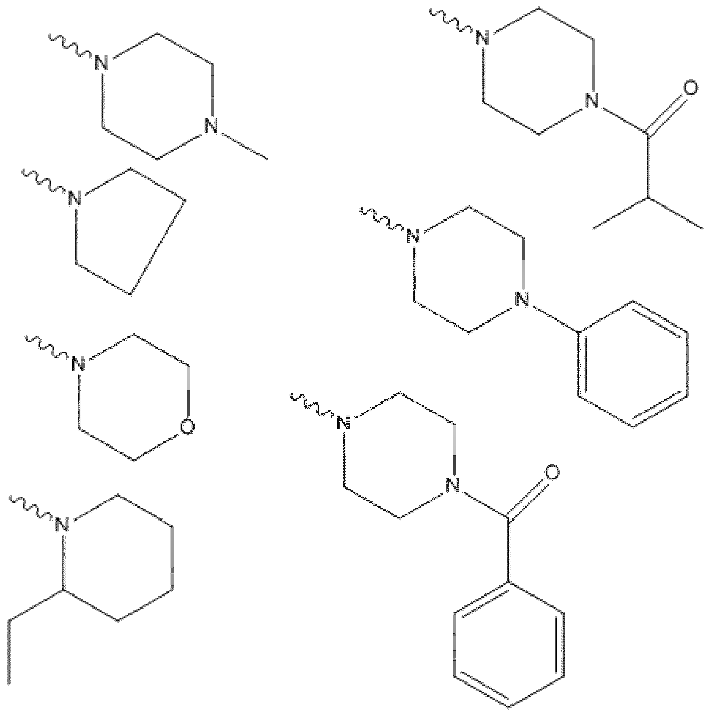

3. The compound according to claim 1 or 2, **characterized in that**:
R¹ is H or (1C-4C)alkyl;
R² is (1C-4C)alkyl, phenyl or a monocyclic aromatic ring having one or more N-, O- or
S- atoms in the ring, which alkyl, phenyl or aromatic ring is optionally substituted with one or more groups selected from a (1C-4C)alkyl or a halogen;
R⁵ and R⁶ are hydrogen.

4. The compound according to any one of claim 1-3, **characterized in that** X is S.

5. The compound according to any one of claim 1-4, **characterized in that** R¹ is H or (1C-4C)alkyl and R² is (1C-4C)alkyl or phenyl.

6. The compound according to any one of claim 1-5, **characterized in that** R³ is hydrogen.

7. The compound according to any one of claim 1-5, **characterized in that** R³ and R⁴ represent both methyl or R³ is hydrogen.

8. The compound according to any one of claims 1-6, **characterized in that** the compound has a structure according to formula II: 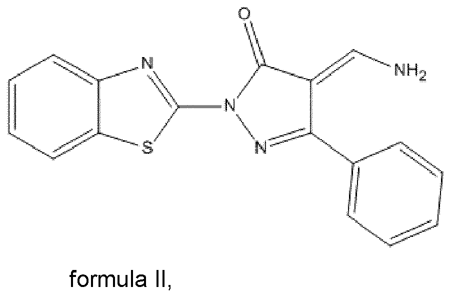 or a pharmaceutically acceptable addition salt thereof.

9. The compound according to any one of claims 1 - 8 for use in a method of therapy.

10. The compound for use according to claim 9, **characterized in that** the method of therapy is a method for treatment or prevention of a carcinoma.

11. The compound for use according to claim 10, **characterized in that** the carcinoma is selected from the group consisting of gastric cancer, bladder cancer, esophageal cancer, breast cancer, prostate cancer and pancreas cancer.

12. The compound for use according to claim 11 wherein the carcinoma is prostate cancer.

13. The compound for use according to any one of claim 9-12, **characterized in that** the method of therapy is directed towards delaying, preventing, treating or reversing metastasis.

14. A pharmaceutical composition comprising a compound according to any one of claims 1 - 8.

15. The pharmaceutical composition according to claim 14, **characterized in that** the composition comprises one or more other compounds with therapeutic effects in treatment of a carcinoma.
